(19) Europäisches Patentamt

European Patent Office

Office européen des brevets

(11) **EP 4 439 584 A1**

(12) **EUROPEAN PATENT APPLICATION**
published in accordance with Art. 153(4) EPC

(43) Date of publication:
**02.10.2024 Bulletin 2024/40**

(21) Application number: **22906035.5**

(22) Date of filing: **12.10.2022**

(51) International Patent Classification (IPC):
***G16H 50/20*** (2018.01)

(52) Cooperative Patent Classification (CPC):
**G16H 50/20; G16H 50/70; G16H 70/60**

(86) International application number:
**PCT/CN2022/124928**

(87) International publication number:
**WO 2023/109283 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.12.2021 CN 202111540631**

(71) Applicants:
• **Huawei Cloud Computing Technologies Co., Ltd.
Gui'an New District, Guizhou 550025 (CN)**
• **GUANGZHOU INSTITUTE OF RESPIRATORY HEALTH
Guangzhou, Guangdong 510120 (CN)**

(72) Inventors:
• **WANG, Yimin
Guangzhou, Guangdong 510120 (CN)**
• **ZHANG, Changzheng
Guizhou 550025 (CN)**
• **TU, Dandan
Guizhou 550025 (CN)**
• **GAO, Yi
Guangzhou, Guangdong 510120 (CN)**
• **ZHENG, Jinping
Guangzhou, Guangdong 510120 (CN)**
• **CHEN, Xiaoshi
Guizhou 550025 (CN)**

(74) Representative: **Körber, Martin Hans
Mitscherlich PartmbB
Patent- und Rechtsanwälte
Karlstraße 7
80333 München (DE)**

(54) **METHOD AND APPARATUS FOR INTERPRETING MEDICAL TEST DATA**

(57) A method and an apparatus for interpreting medical detection data are disclosed. The method includes: obtaining medical detection data to be interpreted, where the medical detection data to be interpreted includes a detection image and indicator data; performing image recognition on the detection image, to obtain image recognition data; and interpreting, by using a rule library, the indicator data and the image recognition data, to obtain an interpretation result, where an interpretation rule in the rule library is obtained by mining and analyzing a plurality of pieces of interpreted data based on a data mining algorithm, and the rule library is dynamically updated with an interpretation process. Because the interpretation rule in the rule library is obtained by mining and analyzing the plurality of pieces of interpreted data based on the data mining algorithm, a potential data law that is difficult to be discovered by human can be mined, and a more accurate interpretation rule can be extracted. In addition, the rule library is dynamically updated with the interpretation process, so that data laws covered by the rule library can be enriched, thereby improving accuracy of automatic interpretation of the medical detection data.

101

102

104

Data obtaining module

Image recognition module

Interpretation module

401: Obtain medical detection data to be interpreted

402: Output a detection image in the medical detection data to be interpreted

403: Perform image recognition, to obtain image recognition data

404: Output the image recognition data

405: Output indicator data and the image recognition data

406: Perform quality control on the indicator data and the image recognition data

407: Interpret the indicator data and the image recognition data, to obtain an interpretation result

FIG. 4

Description

CROSS-REFERENCE TO RELATED APPLICATIONS

This application claims priority to Chinese Patent Application No. 202111540631.9, filed with the China National Intellectual Property Administration on December 16, 2021 and entitled "METHOD AND APPARATUS FOR INTERPRETING MEDICAL DETECTION DATA", which is incorporated herein by reference in its entirety.

TECHNICAL FIELD

This application relates to the field of artificial intelligence, and in particular, to a method and an apparatus for interpreting medical detection data.

BACKGROUND

Medical detection data is an important basis for determining a diagnosis and treatment status of a patient. Accuracy of interpreting the medical detection data is crucial to treatment of the patient. For example, in the field of pulmonary functions, most pulmonary function diseases feature a sudden onset, and an accurate interpretation result needs to be obtained as soon as possible for targeted treatment. Otherwise, the disease is very easy to deteriorate, and is even life-threatening. Therefore, interpretation of the medical detection data requires extremely high accuracy and lower fault tolerance. If the accuracy of the medical detection data is insufficient, an opportunity for early intervention is missed, resulting in an irreversible consequence. However, the medical detection data is complex, mainly in a form of images, and is very easy to be misinterpreted. Accurate interpretation of the medical detection data depends particularly on high-level specialist doctors.

The specialist doctors can accurately determine a diagnosis and treatment status of a patient by interpreting the medical detection data. However, there are currently a large quantity of patients and limited medical resources. To improve diagnosis efficiency, currently, a more common practice is to abstract several fixed interpretation rules based on experience of the specialist doctors for automatic interpretation. However, these interpretation rules are obtained through artificial abstraction based on superficial pathological characteristics, and it is difficult to explore a deep pathological characteristic of the medical detection data. In addition, these interpretation rules are fixed interpretation rules, and accuracy of the automatic interpretation is far lower than accuracy of interpretation by the specialist doctors. Therefore, how to improve accuracy of automatic interpretation of medical detection data is an urgent problem to be resolved.

SUMMARY

This application provides a method and an apparatus for interpreting medical detection data, to improve accuracy of automatic interpretation of medical detection data.

According to a first aspect, this application provides a method for interpreting medical detection data. The method may be performed by an apparatus for interpreting medical detection data provided in this application. The apparatus for interpreting medical detection data may be an electronic device provided in this application. The method includes:

obtaining medical detection data to be interpreted, where the medical detection data to be interpreted includes a detection image and indicator data; performing image recognition on the detection image, to obtain image recognition data; and interpreting, by using a rule library, the indicator data and the image recognition data, to obtain an interpretation result, where an interpretation rule in the rule library is obtained by mining and analyzing a plurality of pieces of interpreted data based on a data mining algorithm, and the rule library is dynamically updated with an interpretation process.

In the foregoing manner, the interpretation rule in the rule library is obtained by mining and analyzing the plurality of pieces of interpreted data based on the data mining algorithm, a potential data law that is difficult to be discovered by human can be mined, and a more accurate interpretation rule can be extracted. In addition, the rule library is dynamically updated with the interpretation process, so that data laws covered by the rule library can be enriched, and the indicator data and the image recognition data can be interpreted more accurately, thereby improving accuracy of automatic interpretation of the medical detection data.

In the method for interpreting medical detection data provided in this application, the rule library may be updated in the following manner:

mining and analyzing the plurality of pieces of interpreted data based on the data mining algorithm, to obtain the interpretation rule, where the interpreted data includes indicator data, image recognition data, and an interpretation result obtained based on the indicator data and the image recognition data; and updating the rule library based on the interpretation rule.

In the foregoing manner, the plurality of pieces of interpreted data are mined and analyzed, so that data characteristics of the indicator data and the image recognition data that correspond to the interpretation result can be learned, and a more accurate interpretation rule can be obtained; and the rule library is updated, to obtain a more accurate rule library.

In a possible design, the interpretation rule may be obtained in the following manner:

classifying the plurality of pieces of interpreted data, to obtain interpreted data of a plurality of categories; selecting, from the interpreted data of the plurality of categories, interpreted data of a first category that meets a preset condition, where the preset condition is that a quantity of similar interpreted data in the interpreted data of the category is greater than a quantity threshold, interpretation results of any two pieces of similar interpreted data are the same, and indicator data of the two pieces of similar interpreted data falls within a same value range and/or image recognition data of the two pieces of similar interpreted data falls within a same value range; and obtaining the interpretation rule based on the similar interpreted data in the interpreted data of the first category.

In the foregoing manner, based on the similar interpreted data in the interpreted data of the first category that meets the preset condition, a commonality of the similar interpreted data may be mined, so that for similar interpreted data of a same category, an interpretation rule for accurate interpretation can be obtained.

In the method for interpreting medical detection data provided in this application, the detection image may be in a plurality of forms. Using a signal wave image as an example, specific steps of the performing image recognition on the detection image, to obtain image recognition data may be as follows:

performing image recognition on the signal wave image, to obtain at least one piece of attribute information of a signal wave waveform in the signal wave image.

Image recognition is performed on the signal wave image, to simplify complex image information into the at least one piece of attribute information, so that interpretation is easy, and the interpretation process is simplified.

In a possible design, after the image recognition data is obtained, quality control may be performed on the indicator data and the image recognition data based on a quality control rule. After it is determined that a preset quality control specification is met, the indicator data and the image recognition data are interpreted by using the rule library, to obtain the interpretation result.

In a possible design, specific steps of the interpreting, by using a rule library, the indicator data and the image recognition data may be as follows:

interpreting, by using the rule library, the image recognition data, to obtain at least two candidate interpretation results; and selecting the interpretation result from the at least two candidate interpretation results based on the indicator data.

In the foregoing manner, the image recognition data may be first interpreted by using the rule library, to narrow a range of a final interpretation result down to the at least two candidate interpretation results, and then a more accurate interpretation result is selected from the at least two candidate interpretation results based on the indicator data, so that the more accurate interpretation result can be obtained.

In a possible design, there are at least two pieces of indicator data; and a specific manner of the selecting the interpretation result from the at least two candidate interpretation results may be as follows:

separately obtaining, based on value intervals of the at least two pieces of indicator data, candidate interpretation results corresponding to the corresponding value intervals, where the value intervals of the at least two pieces of indicator data are divided into a plurality of value intervals, and each value interval corresponds to at least one candidate interpretation result; and selecting, from all candidate interpretation results obtained based on the at least two pieces of indicator data, a candidate interpretation result that appears most frequently, to obtain the interpretation result.

In the foregoing manner, based on the value intervals of the at least two pieces of indicator data, the candidate interpretation result corresponding to the corresponding value interval may be determined, and the candidate interpretation result that appears most frequently is a more accurate interpretation result, so that the more accurate interpretation result can be obtained.

In a possible design, a specific manner of the selecting the interpretation result from the at least two candidate interpretation results may alternatively be as follows:

obtaining target indicator data respectively corresponding to the at least two candidate interpretation results, where the target indicator data corresponding to any one candidate interpretation result is obtained based on indicator data in interpreted data corresponding to the candidate interpretation result; separately determining similarities between the indicator data in the medical detection data and the target indicator data corresponding to the at least two candidate interpretation results; and selecting a candidate interpretation result corresponding to a highest similarity, to obtain the interpretation result.

In the foregoing manner, because the target indicator data corresponding to any one candidate interpretation result is obtained based on the indicator data in the interpreted data corresponding to the candidate interpretation result, the target indicator data corresponding to the candidate interpretation result may represent a common characteristic of the indicator data corresponding to the candidate interpretation result. Therefore, a higher similarity between the indicator data and the target indicator data indicates that the indicator data is more compliant with a data characteristic of the indicator data corresponding to the candidate interpretation result, and the interpretation result is more accurate. In this way, the more accurate interpretation result can be obtained.

According to a second aspect, this application provides an apparatus for interpreting medical detection data. The apparatus may be an electronic device provided in this application. The apparatus includes:

a data obtaining module, configured to obtain medical detection data to be interpreted, where the medical detection data to be interpreted includes a detection image and indicator data;
an image recognition module, configured to perform image recognition on the detection image, to obtain image recognition data; and
an interpretation module, configured to interpret, by using a rule library, the indicator data and the image recognition data, to obtain an interpretation result, where an interpretation rule in the rule library is obtained by mining and analyzing a plurality of pieces of interpreted data based on a data mining algorithm, and the rule library is dynamically updated with an interpretation process.

In a possible design, the apparatus further includes a data mining module. The data mining module is configured to:

mine and analyze the plurality of pieces of interpreted data based on the data mining algorithm, to obtain the interpretation rule, where the interpreted data includes indicator data, image recognition data, and an interpretation result obtained based on the indicator data and the image recognition data; and update the rule library based on the interpretation rule.

In a possible design, the data mining module is specifically configured to:

classify the plurality of pieces of interpreted data, to obtain interpreted data of a plurality of categories; select, from the interpreted data of the plurality of categories, interpreted data of a first category that meets a preset condition, where the preset condition is that a quantity of similar interpreted data in the interpreted data of the category is greater than a quantity threshold, interpretation results of any two pieces of similar interpreted data are the same, and indicator data of the two pieces of similar interpreted data falls within a same value range and/or image recognition data of the two pieces of similar interpreted data falls within a same value range; and obtain the interpretation rule based on the similar interpreted data in the interpreted data of the first category.

In a possible design, the detection image includes a signal wave image, and the image recognition module is specifically configured to:

perform image recognition on the signal wave image, to obtain at least one piece of attribute information of a signal wave waveform in the signal wave image.

In a possible design, the interpretation module is specifically configured to:

interpret, by using the rule library, the image recognition data, to obtain at least two candidate interpretation results; and select the interpretation result from the at least two candidate interpretation results based on the indicator data.

In a possible design, there are at least two pieces of indicator data; and the interpretation module is specifically configured to:

separately obtain, based on value intervals of the at least two pieces of indicator data, candidate interpretation results corresponding to the corresponding value intervals, where the value intervals of the at least two pieces of indicator data are divided into a plurality of value intervals, and each value interval corresponds to at least one candidate interpretation result; and select, from all candidate interpretation results obtained based on the at least two pieces of indicator data, a candidate interpretation result that appears most frequently, to obtain the interpretation result.

In a possible design, the interpretation module is specifically configured to:

obtain target indicator data respectively corresponding to the at least two candidate interpretation results, where the target indicator data corresponding to any one candidate interpretation result is obtained based on indicator data in interpreted data corresponding to the candidate interpretation result; separately determine similarities between the indicator data in the medical detection data and the target indicator data corresponding to the at least two candidate interpretation results; and select a candidate interpretation result corresponding to a highest similarity, to obtain the interpretation result.

In a possible design, before interpreting, by using the rule library, the indicator data and the image recognition data, to obtain the interpretation result, the interpretation module is further configured to:

determine, based on a quality control rule, that the indicator data and the image recognition data meet a preset quality control specification.

According to a third aspect, this application provides an electronic device. The electronic device includes one or more processors and one or more memories. The one or more memories store one or more computer instructions. When the one or more computer instructions are executed by the one or more processors, the electronic device is enabled to perform the method according to any one of the first aspect.

According to a fourth aspect, this application provides a computer-readable storage medium. The computer-readable storage medium includes computer instructions. When the computer instructions are run on a computer, the computer is enabled to perform the method according to any one of the first aspect.

According to a fifth aspect, this application provides a computer program product. When a computer reads and executes the computer program product, the computer is enabled to perform the method according to any one of the first aspect.

For beneficial effects of the second aspect to the fifth aspect, refer to the beneficial effects of the first aspect. Details are not described again.

## BRIEF DESCRIPTION OF DRAWINGS

FIG. 1 is a schematic diagram of an applicable architecture for constructing or updating a rule library of medical detection data according to an embodiment of this application;

FIG. 2 is a schematic diagram of an applicable architecture of a method for interpreting medical detection data according to an embodiment of this application;

FIG. 3 is a schematic flowchart of a method for updating a rule library of medical detection data according to an embodiment of this application;
FIG. 4 is a schematic flowchart of a method for interpreting medical detection data according to an embodiment of this application;
FIG. 5 is a schematic diagram of a structure of an apparatus for interpreting medical detection data according to an embodiment of this application; and
FIG. 6 is a schematic diagram of a structure of an electronic device according to an embodiment of this application.

## DESCRIPTION OF EMBODIMENTS

The following clearly and completely describes the technical solutions in embodiments of this application with reference to the accompanying drawings in embodiments of this application.

Terms used in the following embodiments are merely intended to describe specific embodiments, but are not intended to limit this application. The terms "one", "a", "the", "the foregoing", "this", and "the one" in singular forms used in the specification and the appended claims of this application are also intended to include forms such as "one or more", unless otherwise specified in the context clearly. It should be further understood that, in embodiments of this application, "one or more" refers to one or more than two (including two); and "and/or" describes an association relationship between associated objects, and indicates that three relationships may exist. For example, A and/or B may indicate the following cases: Only A exists, both A and B exist, and only B exists, where A and B may be singular or plural. The character "/" generally indicates an "or" relationship between the associated objects.

Reference to "one embodiment" or "some embodiments" described in the specification means that a specific characteristic, structure or feature described in combination with this embodiment is included in one or more embodiments of this application. Therefore, statements such as "in an embodiment", "in some embodiments", "in some other embodiments", and "in other embodiments" that appear in different places in the specification do not necessarily mean referring to a same embodiment. Instead, the statements mean "one or more but not all of embodiments", unless otherwise specifically emphasized in another manner. The terms "include", "comprise", "have", and their variants all mean "include but are not limited to", unless otherwise specifically emphasized in another manner.

In embodiments of this application, the term "a plurality of means two or more. In view of this, in embodiments of this application, "a plurality of may also be understood as "at least two". "At least one" may be understood as one or more, for example, one, two, or more. For example, including at least one means including one, two, or more, and there is no limitation on which is included. For example, if at least one of A, B, and C is included, A, B, C, A and B, A and C, B and C, or A, B, and C may be included. Similarly, understandings of descriptions such as "at least one" are also similar to this. The term "and/or" describes an association relationship between associated objects, and indicates that three relationships may exist. For example, A and/or B may indicate the following cases: Only A exists, both A and B exist, and only B exists. In addition, the character "/", unless otherwise specified, generally indicates an "or" relationship between the associated objects.

Unless otherwise specified, ordinal numbers such as "first" and "second" in embodiments of this application are used to distinguish between a plurality of objects, and are not intended to limit a sequence, a time sequence, priorities, or importance of the plurality of objects.

For ease of understanding, terms in embodiments of this application are explained and described, and explanations and descriptions of the terms are also used as a part of invention content of embodiments of this application.

## Application scenario

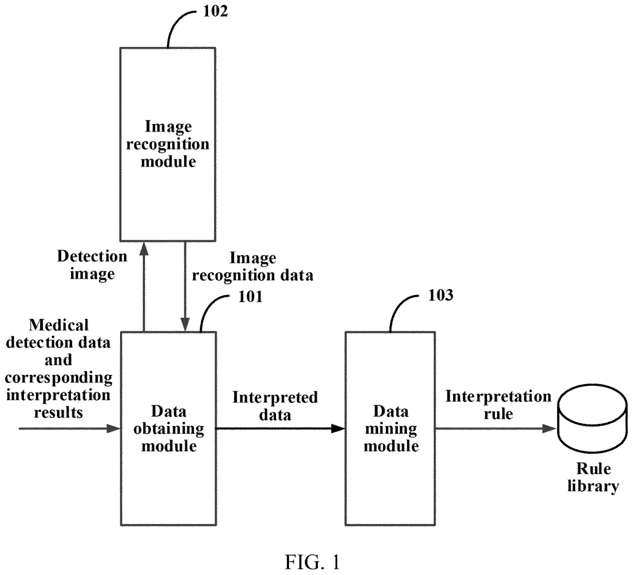

FIG. 1 is a schematic diagram of an applicable architecture for constructing or updating a rule library of medical detection data according to an embodiment of this application. The architecture may include a data obtaining module 101, an image recognition module 102, and a data mining module 103. The foregoing modules may be implemented by software or hardware. For example, the foregoing modules are all deployed in one device and are implemented by software. The foregoing modules may alternatively be implemented by one device, or may be implemented by a plurality of devices in cooperation. Alternatively, some modules may be implemented by software, and the other modules may be implemented by hardware, and may be deployed in a cloud, an edge end, a terminal device, or the like. This is not limited herein.

Functions of the modules in the architecture shown in FIG. 1 may be specifically as follows:

The data obtaining module 101 is configured to: obtain a plurality of pieces of medical detection data and corresponding interpretation results, and output detection images in the medical detection data to the image recognition module 102.

Any piece of medical detection data may include indicator data and a detection image. There may be one or more pieces of indicator data. A form of the indicator data is unidimensional data such as a blood oxygen saturation level or a body temperature. A type of the detection image is not limited herein, and is for example, a signal wave image or a lung image. The signal wave image may be a cough wave image, an electrocardiogram image, or the like. A case in which the medical detection data includes the indicator data and the detection image is only an example case to which the data obtaining module 101 in FIG. 1 is applied. In some embodiments, the medical detection data may be the indicator data, the detection image, or the like.

The image recognition module 102 is configured to: perform image recognition on the input detection image, to obtain image recognition data, and output the image recognition data to the data obtaining module 101.

The data obtaining module 101 is further configured to obtain, from the image recognition module 102, the image recognition data corresponding to the detection image. Therefore, in the data obtaining module 101, one piece of interpreted data may be formed based on the indicator data of any piece of medical detection data, the image recognition data corresponding to the detection image, and the interpretation result of the medical detection data. In this way, the data obtaining module 101 may obtain a plurality of pieces of interpreted data based on the plurality of pieces of medical detection data. The data obtaining module 101 is further configured to output the plurality of pieces of interpreted data to the data mining module 103.

It should be noted that a process of forming one piece of interpreted data may alternatively be as follows: For any piece of medical detection data, the image recognition module 102 outputs the image recognition data in the medical detection data to the data mining module 103, and the data obtaining module 101 outputs the indicator data and the interpretation result of the medical detection data to the data mining module 103. In this way, in the data mining module 103, one piece of interpreted data may be formed based on the indicator data, the image recognition data, and the interpretation result of the medical detection data.

The data mining module 103 is configured to: perform quality control on the plurality of pieces of input interpreted data, and delete interpreted data that does not meet a preset quality control specification.

The quality control refers to determining quality of data, to determine whether the data meets the preset quality control specification. For example, for indicator data in the interpreted data, a body temperature is used as an example. If a preset quality control specification of a body temperature of a patient is that a value of the body temperature ranges from 35 degrees to 40 degrees, when the body temperature of the patient falls beyond the value range, for example, the body temperature of the patient is 20 degrees, it is determined that the preset quality control specification is not met, indicating that the indicator data is collected incorrectly. For image recognition data in the interpreted data, image recognition data of a respiratory waveform (respiratory waveform) image is used as an example. If a preset quality control specification of breathing duration of the respiratory waveform image is that the breathing duration is greater than preset duration, when the breathing duration in the image recognition data is less than the preset duration, it may be determined that the preset quality control specification is not met.

For any piece of interpreted data, the data mining module 103 may use, after the interpreted data succeeds in the quality control, the interpreted data as interpreted data that participates in data mining and analyzing, so that invalid interpreted data in the data mining module 103 can be reduced, thereby improving effect of data mining and analyzing. In some embodiments, the data mining module 103 may not perform quality control on the input interpreted data, but directly use the input interpreted data as interpreted data that participates in data mining and analyzing.

The data mining module 103 is further configured to: mine and analyze, based on a data mining algorithm, a plurality of pieces of interpreted data that meet the preset quality control specification, to obtain an interpretation rule; and update the rule library based on the interpretation rule. It should be noted that the data mining algorithm used for data mining and analyzing in the data mining module 103 is not limited herein, and may be, for example, a support vector machine algorithm, a decision tree algorithm, or a naive Bayes algorithm.

It should be noted that a parameter in the interpretation rule may be dynamically changed and updated, and a value of the parameter in the interpretation rule in an initial rule library is set to a default value. For example, for any first interpretation rule in the rule library, the first interpretation rule is that if a proportion of a quantity of respiratory cycles of respiratory distress counted to a total quantity of respiratory cycles in the image recognition data is greater than x, the interpretation result corresponding to the medical detection data is dyspnea, where x is the parameter in the interpretation rule. In the initial rule library, x may be a default value, for example, 30%.

There are a plurality of specific implementations in which the data mining module 103 updates the rule library. For example, the data mining module 103 changes the first interpretation rule in the rule library to a second interpretation rule. In the first interpretation rule, x is set to 30%, and in the first interpretation rule, x is set to 28%. In some examples, the rule library may not include the first interpretation rule. The data mining module 103 adds the first interpretation rule to the rule library.

It should be noted that the architecture shown in FIG. 1 is only an example, and components in the architecture may be flexibly added or deleted based on a specific status. For example, the data obtaining module 101 may alternatively directly obtain a plurality of pieces of interpreted data from the outside, and output the plurality of pieces of interpreted data to the data mining module 103; or when the medical detection data is indicator data, after obtaining the indicator data, the data obtaining module 101 outputs the indicator data to the data mining module 103. In these cases, the architecture may not include the image recognition module 102.

The dynamically updated rule library in the architecture shown in FIG. 1 may be used for automatic interpretation of the medical detection data.

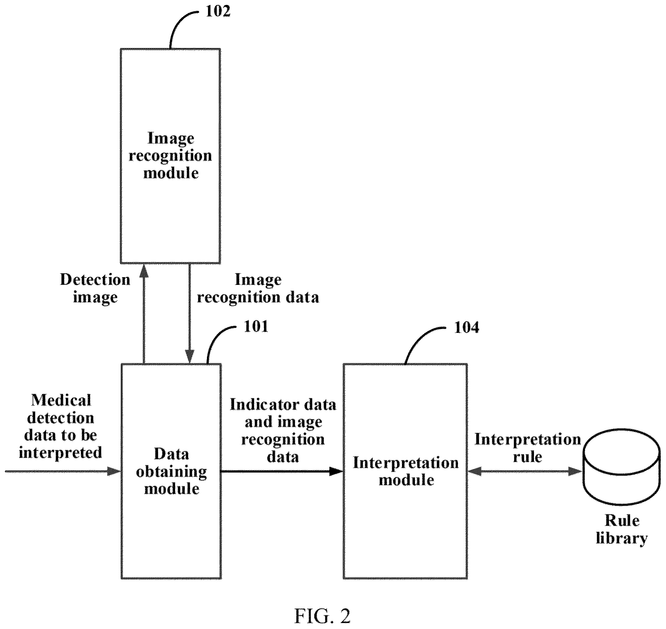

FIG. 2 is a schematic diagram of an applicable architecture of a method for interpreting medical detection data according to an embodiment of this application. The architecture may include a data obtaining module 101, an image recognition module 102, and an interpretation module 104. The foregoing modules may be implemented by software or hardware. For example, the foregoing modules are all deployed in one device and are implemented by software. Alternatively, some modules may be implemented by software, and the other modules may be implemented by hardware. The foregoing modules may alternatively be deployed in one device, or may be implemented by a plurality of devices in cooperation. This is not limited herein.

Functions of the modules in the architecture shown in FIG. 2 may be specifically as follows:
The data obtaining module 101 is configured to obtain medical detection data to be interpreted.

The medical detection data to be interpreted may include indicator data and a detection image. This case is only an example case to which the data obtaining module 101 in FIG. 2 is applied. In some embodiments, the medical detection data may be the indicator data, the detection image, or the like.

The data obtaining module 101 is further configured to output the detection image in the medical detection data to the image recognition module 102.

The image recognition module 102 is configured to: perform image recognition on the input detection image, to obtain image recognition data, and output the image recognition data to the data obtaining module 101.

The data obtaining module 101 is further configured to: obtain, from the image recognition module 102, the image recognition data corresponding to the detection image, and output the indicator data and the image recognition data to the interpretation module 104.

In some cases, the image recognition module 102 may alternatively output the image recognition data to the interpretation module 104.

The interpretation module 104 is configured to perform quality control on the medical detection data.

The interpretation module 104 may interpret, after the medical detection data succeeds in the quality control, the indicator data and the image recognition data by using a rule library. In this way, invalid operations of the interpretation module 104 can be reduced. The interpretation module 104 may alternatively directly interpret the input medical detection data without performing quality control on the medical detection data.

The interpretation module 104 is configured to interpret the indicator data and the image recognition data by using the rule library, to obtain an interpretation result. For example, the image recognition data may be interpreted by using the rule library, to obtain one target interpretation result or at least two candidate interpretation results. When obtaining the at least two candidate interpretation results, the target interpretation result may be selected from the at least two candidate interpretation results based on the indicator data. In another case, the indicator data may alternatively be interpreted by using the rule library, to obtain at least two candidate interpretation results. The target interpretation result is selected from the at least two candidate interpretation results based on the image recognition data.

It should be noted that the architecture shown in FIG. 2 is only an example, and components in the architecture for implementing the method for interpreting medical detection data may be flexibly added or deleted based on a specific status. For example, when the medical detection data is the indicator data, after obtaining the indicator data, the data obtaining module 101 may directly output the indicator data to the interpretation module 104. In this case, the architecture may not include the image recognition module 102.

In some cases, the architecture shown in FIG. 1 and the architecture shown in FIG. 2 may be a same architecture. For example, the architecture may include the data obtaining module 101, the image recognition module 102, the data mining module 103, and the interpretation module 104. In other words, the architecture may be used to update the rule library and interpret the medical detection data based on the rule library. The following describes in detail a method that can be performed in the architecture shown in FIG. 1 or FIG. 2.

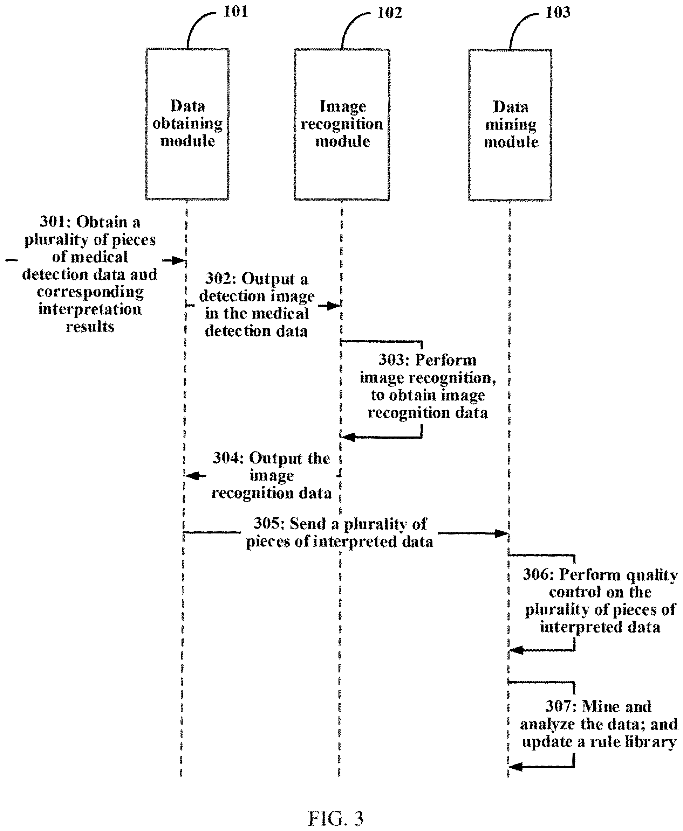

FIG. 3 is a schematic flowchart of a method for updating a rule library of medical detection data according to an embodiment of this application. The method shown in FIG. 3 may be performed in the architecture shown in FIG. 1. Specific steps may be as follows:

Step 301: The data obtaining module 101 obtains a plurality of pieces of medical detection data and corresponding interpretation results.

The medical detection data and the interpretation result may be specifically medical detection data and an interpretation result of a confirmed patient. In this setting, a data characteristic of the medical detection data of the confirmed patient may be learned, so that the data obtaining module 101 is more sensitive to the medical detection data of the patient, and an accurate interpretation result is more easily obtained.

In the process shown in FIG. 3, an example in which the medical detection data includes indicator data and a detection image is used. The detection image may be specifically a signal wave image. For example, the signal wave image is a respiratory waveform image, and the respiratory waveform image may be specifically a volume-time curve. The indicator data includes at least one indicator. Using a lung function indicator as an example, the indicator may be a vital capacity (vital capacity, VC), a total lung capacity (total lung capacity, TCL), a maximal voluntary ventilation (maximal voluntary ventilation, MVV), or the like. Examples are not listed herein one by one.

Step 302: The data obtaining module 101 outputs the detection image in the medical detection data to the image recognition module 102.

Step 303: The image recognition module 102 performs image recognition on the input detection image, to obtain image recognition data.

A method for performing image recognition on the input detection image by the image recognition module 102 may be implemented by using a neural network such as a convolutional neural network (convolutional neural network, CNN) or a support vector machine (support vector machine, SVM), and may use a deep learning algorithm. This is not limited herein. The detection image may include a signal wave image. The image recognition data may be specifically attribute information of a signal wave waveform in the signal wave image, for example, at least one piece of attribute information of a respiratory waveform image, such as respiratory distress information, inhaled tidal volume information, and exhaled tidal volume information.

Step 304: The image recognition module 102 outputs the image recognition data to the data obtaining module 101.

After step 304 is performed, in the data obtaining module 101, one piece of interpreted data may be formed based on indicator data of any piece of medical detection data, image recognition data corresponding to a detection image, and an interpretation result of the medical detection data.

Step 305: The data obtaining module 101 sends a plurality of pieces of interpreted data to the data mining module 103.

Step 306: The data mining module 103 performs quality control on the plurality of pieces of interpreted data, and deletes interpreted data that does not meet a preset quality control specification.

Step 307: The data mining module 103 mines and analyzes, based on a data mining algorithm, a plurality of pieces of interpreted data that meet the preset quality control specification, to obtain an interpretation rule; and updates a rule library based on the interpretation rule.

Step 307 may be performed when a first preset condition is met.

The first preset condition may be that accumulated duration from a preset moment to a moment at which the data mining module 103 receives the plurality of pieces of interpreted data is greater than or equal to preset duration. The first preset condition may alternatively be that a total quantity of interpreted data accumulatively obtained by the data mining module 103 is greater than a first threshold. The first preset condition may alternatively be that a total quantity of interpreted data accumulatively obtained by the data mining module 103 in a preset time period is greater than a second threshold. The first preset condition may be flexibly set based on a specific status. This is not limited herein.

The process of obtaining the interpretation rule in step 307 may be specifically as follows:
classifying the plurality of pieces of interpreted data, to obtain interpreted data of a plurality of categories; selecting, from the interpreted data of the plurality of categories, interpreted data of a first category that meets a second preset condition; and obtaining the interpretation rule based on the interpreted data of the first category.

The second preset condition is that a quantity of similar interpreted data in the interpreted data of the category is greater than a quantity threshold, interpretation results of any two pieces of similar interpreted data are the same, and indicator data of the two pieces of similar interpreted data falls within a same value range, and/or image recognition data of the two pieces of similar interpreted data falls within a same value range.

For example, when the rule library is generated, the first preset condition may be that the accumulated duration from the preset moment is greater than or equal to the preset duration. For example, if the preset moment is 18 o'clock on January 1, after receiving a plurality of pieces of interpreted data at 19 o'clock on January 8, the data mining module 103 may mine and analyze data based on all the interpreted data (including a plurality of pieces of interpreted data that are input this time) received between 18 o'clock on January 1 and 19 o'clock on January 8, to obtain a mining and analyzing result; and update the rule library based on the mining and analyzing result. A specific process may be as follows:

A first interpretation rule in the rule library is used as an example. The first interpretation rule at 18 o'clock on January 1 may be specifically that if a proportion of a quantity of respiratory cycles of respiratory distress counted to a total quantity of respiratory cycles in the image recognition data is greater than x, the interpretation result corresponding to the medical detection data is dyspnea, where x is a parameter in the first interpretation rule. In this case, it is assumed that x is 30%, and a range is [10%, 60%].

After receiving the plurality of pieces of interpreted data at 19 o'clock on January 8, the data mining module 103 may classify, by using a clustering algorithm, all the interpreted data received between 18 o'clock on January 1 and 19 o'clock on January 8. A classification result includes interpreted data of a plurality of categories, and interpreted data of the first category may be first selected from the interpreted data of the plurality of categories.

The second preset condition is that the quantity of similar interpreted data in the interpreted data of the category is greater than the quantity threshold. It should be noted that the quantity threshold may be fixed, or may be obtained based on a total quantity of interpreted data of the category. For example, a total quantity of interpreted data of a category is 10000. Similar interpreted data in the interpreted data of this category meets a condition that a quantity of respiratory cycles of respiratory distress accounts for more than 30% of a total quantity of respiratory cycles, and an interpretation result is "dyspnea". A quantity of similar interpreted data is 9000. The quantity threshold is a product of a preset proportion and the total quantity of interpreted data of this category, and the preset proportion is 85%. In this case, the quantity threshold is 8500, and this category is the first category.

Further, the interpretation rule may be obtained based on the interpreted data of the first category. For example, statistical analysis is performed on the similar interpreted data in the interpreted data of the first category, and the interpretation rule is obtained based on a statistical analysis result.

For example, the parameter x in the first interpretation rule may be updated based on the preset proportion and the proportion of the quantity of similar interpreted data in the interpreted data of the first category, to obtain a second interpretation rule. For example, the parameter x may be updated based on the following formula:

$$X1-X2=(C2-C1)*k.$$

X1 is a value of the parameter x in the first interpretation rule, X2 is a value of the parameter x in the second interpretation rule, C 1 is the proportion of the quantity of similar interpreted data in the interpreted data of the first category, C2 is the preset proportion, and k is a coefficient.

If k is set to 0.4, in the formula, X1 is 30%, C1 is 90%, C2 is 85%, and it may be learned that X2 is 28%. In this case, the parameter x in the first interpretation rule is updated to 28%, and the first interpretation rule is changed as follows: if the proportion of the quantity of respiratory cycles of respiratory distress counted to the total quantity of respiratory cycles in the image recognition data is greater than 28%, the interpretation result corresponding to the medical detection data is dyspnea. In addition, the preset proportion C2 of the first interpretation rule may be further updated to C1.

In the method shown in FIG. 3, a case in which the medical detection data includes the indicator data and the detection image is shown, and there may be another case. For example, the data obtaining module 101 obtains a plurality of pieces of interpreted data, and outputs the plurality of pieces of interpreted data to the data mining module 103 for data mining and analyzing. For a method process in another case, refer to the foregoing descriptions. Details are not described herein again.

The dynamically updated rule library in the method process shown in FIG. 3 may be used for automatic interpretation of the medical detection data.

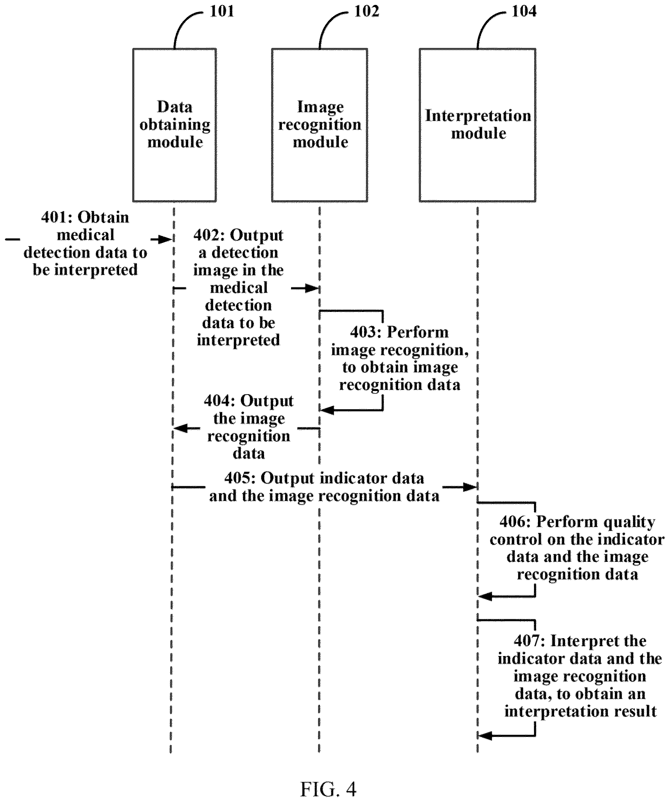

FIG. 4 is a schematic flowchart of a method for interpreting medical detection data according to an embodiment of this application. The method shown in FIG. 4 may be performed in the architecture shown in FIG. 2. Specific steps may be as follows:

Step 401: The data obtaining module 101 obtains medical detection data to be interpreted.

The medical detection data to be interpreted may include indicator data and a detection image. This case is only an example case to which FIG. 4 is applied.

In some embodiments, the medical detection data may be the indicator data, the detection image, or the like.

Step 402: The data obtaining module 101 outputs the detection image in the medical detection data to be interpreted to the image recognition module 102.

Step 403: The image recognition module 102 performs image recognition on the input detection image, to obtain image recognition data.

The detection image may include a signal wave image. The image recognition data may be specifically at least one piece of attribute information of a signal wave waveform in the signal wave image.

Step 404: The image recognition module 102 outputs the image recognition data to the data obtaining module 101.

Step 405: The data obtaining module 101 outputs the indicator data and the image recognition data from the image recognition module 102 to the interpretation module 104.

Step 406: The interpretation module 104 performs quality control on the indicator data and the image recognition data, and determines whether the indicator data and the image recognition data meet a preset quality control specification.

If yes, step 407 is performed; otherwise, the process is ended.

Step 407: Interpret the indicator data and the image recognition data by using a rule library, to obtain an interpretation result.

An implementation (a first implementation) of step 407 may be as follows:

interpreting, by using the rule library, the image recognition data, to obtain at least two candidate interpretation results; and selecting a target interpretation result from the at least two candidate interpretation results based on the indicator data.

An implementation (a second implementation) of step 407 may alternatively be as follows:

interpreting, by using the rule library, the indicator data, to obtain at least two candidate interpretation results; and selecting a target interpretation result from the at least two candidate interpretation results based on the image recognition data.

It should be noted that the at least two candidate interpretation results may be interpretation results of a series, and one individual candidate interpretation result in the interpretation results is a more accurate interpretation result. For example, the at least two candidate interpretation results are all interpretation results of dyspnea, and specifically include: pulmonary dyspnea, cardiac dyspnea, and hematogenous dyspnea. If the indicator data is further combined, a range may be further narrowed down from the at least two candidate interpretation results, and one candidate interpretation result is selected from the at least two candidate interpretation results as the target interpretation result. For example, a specific type of dyspnea in pulmonary dyspnea, cardiac dyspnea, and hematogenous dyspnea may be determined based on a vital capacity, a heartbeat frequency, and a blood routine indicator.

Implementation steps of the foregoing two implementations are similar. The following uses the first implementation as an example for description. The first implementation may be performed in the following two manners:

Execution manner (1):

For a value of any one of at least two indicators, a candidate interpretation result corresponding to an interval of the value of the indicator is determined; and a candidate interpretation result corresponding to the at least two candidate interpretation results and having a largest quantity of indicator items is used as the target interpretation result.

Based on value intervals of at least two pieces of indicator data, candidate interpretation results corresponding to the corresponding value interval are separately obtained; and a candidate interpretation result that appears most frequently is selected from all candidate interpretation results obtained based on the at least two pieces of indicator data, to obtain the target interpretation result.

The value intervals of the at least two pieces of indicator data are divided into a plurality of value intervals, and each value interval corresponds to at least one candidate interpretation result.

For example, the indicator data has three indicators in total, including a first indicator, a second indicator, and a third indicator. A value of the first indicator falls within a first interval, a value of the second indicator falls within a second interval, and a value of the third indicator falls within a third interval. There are three candidate interpretation results in total, including a first candidate interpretation result, a second candidate interpretation result, and a third candidate interpretation result. The first interval hits the first candidate interpretation result and the third candidate interpretation result, the second interval hits the second candidate interpretation result, and the third interval hits the third candidate interpretation result. Clearly, the first candidate interpretation result appears once, the second candidate interpretation result appears once, and the third candidate interpretation result appears twice. Therefore, the third candidate interpretation result is used as the target interpretation result.

Execution manner (2):

Target indicator data respectively corresponding to the at least two candidate interpretation results is obtained; similarities between the indicator data in the medical detection data and the target indicator data corresponding to the at least two candidate interpretation results are separately determined; and a candidate interpretation result corresponding to a highest similarity is selected, to obtain the target interpretation result.

The target indicator data corresponding to any one candidate interpretation result is obtained based on indicator data in interpreted data corresponding to the candidate interpretation result. For example, the target indicator data may be an average value of the indicator data in the interpreted data corresponding to the candidate interpretation result.

It should be noted that, in a process of calculating the similarities, the indicator data in the medical detection data to be interpreted and the target indicator data may be in a plurality of forms. For example, the indicator data in the medical detection data to be interpreted may be in a form of a medical indicator vector. A value of each dimension of the medical indicator vector is a value of one indicator. The target indicator data corresponding to each of the at least two candidate interpretation results may be in a form of a target medical vector. A value of each dimension of the target medical vector is a target value of one indicator. A manner of setting the target value is not limited. For example, because each candidate interpretation result has a value range corresponding to each indicator, the value of each dimension of the target medical vector may be a median value in each indicator value range.

For example, the medical indicator vector is $P0(x01, ..., x0n)$, where n is a quantity of indicators. Target medical vectors corresponding to the at least two candidate interpretation results include: $P1(x11, ..., x1n)$, $P2(x21, ..., x2n)$, ..., and $Pk(xk1, ..., xkn)$, where x represents an indicator, k is a quantity of the at least two candidate interpretation results, and n is a quantity of medical indicators. For a target medical vector corresponding to any candidate interpretation result, a reciprocal of a Euclidean distance between the target medical vector and the medical indicator vector may be used as a similarity, for example, D1, D2, ..., or Dn, where Di represents a similarity between a medical indicator vector corresponding to the medical indicator data and a target medical vector corresponding to an $i^{th}$ candidate interpretation result. Therefore, a candidate interpretation result corresponding to a highest similarity in D1 to Dn may be used as the target interpretation result, that is, a candidate interpretation result corresponding to a closest target medical vector.

An implementation (a third implementation) of step 407 may alternatively be as follows:
determining, based on the image recognition data and/or the indicator data and the rule library, at least two candidate interpretation results, and combining the at least two candidate interpretation results into a target interpretation result.

For example, if the at least two candidate interpretation results are specifically pulmonary dyspnea, cardiac dyspnea, and hematogenous dyspnea, the at least two candidate interpretation results are combined, to obtain an interpretation result of dyspnea, and the target interpretation result is dyspnea. The combination may be implemented through public keyword matching or in another manner.

In the method shown in FIG. 4, a case in which the medical detection data includes the indicator data and the detection image is shown, and there may be another case. For example, when the medical detection data is the indicator data, the at least two candidate interpretation results may be determined based on the indicator data, and the at least two candidate interpretation results are combined into the target interpretation result. For a method process in another case, refer to the foregoing descriptions. Details are not described herein again.

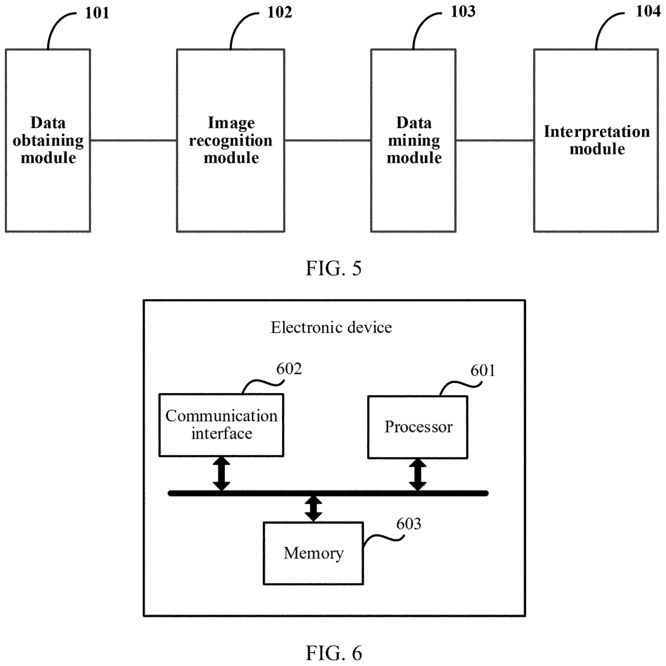

As shown in FIG. 5, an embodiment of this application provides an apparatus for interpreting medical detection data. The apparatus may implement the architecture in FIG. 1 or FIG. 2. The apparatus specifically includes:

> a data obtaining module 101, configured to obtain medical detection data to be interpreted, where the medical detection data to be interpreted includes a detection image and indicator data;
> an image recognition module 102, configured to perform image recognition on the detection image, to obtain image recognition data; and
> an interpretation module 104, configured to interpret, by using a rule library, the indicator data and the image recognition data, to obtain an interpretation result, where an interpretation rule in the rule library is obtained by mining and analyzing a plurality of pieces of interpreted data based on a data mining algorithm, and the rule library is dynamically updated with an interpretation process.

In a possible design, the apparatus further includes a data mining module 103. The data mining module 103 is configured to:
mine and analyze the plurality of pieces of interpreted data based on the data mining algorithm, to obtain the interpretation rule, where the interpreted data includes indicator data, image recognition data, and an interpretation result obtained based on the indicator data and the image recognition data; and update the rule library based on the interpretation rule.

In a possible design, the data mining module 103 is specifically configured to:
classify the plurality of pieces of interpreted data, to obtain interpreted data of a plurality of categories; select, from the interpreted data of the plurality of categories, interpreted data of a first category that meets a preset condition, where the preset condition is that a quantity of similar interpreted data in the interpreted data of the category is greater than a quantity threshold, interpretation results of any two pieces of similar interpreted data are the same, and indicator data of the two pieces of similar interpreted data falls within a same value range and/or image recognition data of the two pieces of similar interpreted data falls within a same value range; and obtain the interpretation rule based on the similar interpreted data in the interpreted data of the first category.

In a possible design, the detection image includes a signal wave image, and the image recognition module 102 is specifically configured to:
perform image recognition on the signal wave image, to obtain at least one piece of attribute information of a signal wave waveform in the signal wave image.

In a possible design, the interpretation module 104 is specifically configured to:
interpret, by using the rule library, the image recognition data, to obtain at least two candidate interpretation results; and select the interpretation result from the at least two candidate interpretation results based on the indicator data.

In a possible design, there are at least two pieces of indicator data; and the interpretation module 104 is specifically configured to:
separately obtain, based on value intervals of the at least two pieces of indicator data, candidate interpretation results corresponding to the corresponding value intervals, where the value intervals of the at least two pieces of indicator data are divided into a plurality of value intervals, and each value interval corresponds to at least one candidate interpretation result; and select, from all candidate interpretation results obtained based on the at least two pieces of indicator data, a candidate interpretation result that appears most frequently, to obtain the interpretation result.

In a possible design, the interpretation module 104 is specifically configured to:
obtain target indicator data respectively corresponding to the at least two candidate interpretation results, where the target indicator data corresponding to any one candidate interpretation result is obtained based on indicator data in interpreted data corresponding to the candidate interpretation result; separately determine similarities between the indicator data in the medical detection data and the target indicator data corresponding to the at least two candidate interpretation results; and select a candidate interpretation result corresponding to a highest similarity, to obtain the interpretation result.

In a possible design, before interpreting, by using the rule library, the indicator data and the image recognition data, to obtain the interpretation result, the interpretation module 104 is further configured to:
determine, based on a quality control rule, that the indicator data and the image recognition data meet a preset quality control specification.

An embodiment of this application further provides an electronic device. The electronic device may have a structure shown in FIG. 6. The electronic device may be a computer device, or may be a chip or a chip system that can support a computer device in implementing the foregoing method.

The electronic device shown in FIG. 6 may include at least one processor 601. The at least one processor 601 is configured to be coupled to a memory, and read and execute instructions in the memory, to implement the steps of the method for interpreting medical detection data provided in embodiments of this application. Optionally, the electronic device may further include a communication interface 602, configured to support the electronic device in receiving or sending signaling or data. The communication interface 602 in the electronic device may be configured to interact with another electronic device. The processor 601 may be configured to implement the steps of the method for interpreting medical detection data performed by the electronic device.

Optionally, the electronic device may further include a memory 603. The memory 603 stores computer instructions. The memory 603 may be coupled to the processor 601 and/or the communication interface 602, and is configured to support the processor 601 in invoking the computer instructions in the memory 603 to implement the steps of the method for interpreting medical detection data. In addition, the memory 603 may be further configured to store data in method embodiments of this application. For example, the memory 603 is configured to store data and instructions that are required for supporting interaction of the communication interface 602, and/or is configured to store configuration information required for the electronic device to perform the method in embodiments of this application.

An embodiment of this application further provides a computer-readable storage medium. The computer-readable storage medium stores computer instructions. When the computer instructions are invoked and executed by a computer, the computer may be enabled to complete the method in any one of the foregoing method embodiments or the possible designs of the foregoing method embodiments. In this embodiment of this application, the computer-readable storage medium is not limited. For example, the computer-readable storage medium may be a RAM (random access memory, random access memory) or a ROM (read-only memory, read-only memory).

This application provides a computer program product. When a computer reads and executes the computer program product, the computer may be enabled to perform the method in any one of the foregoing method embodiments or the possible implementations of the foregoing method embodiments.

All or some of the foregoing embodiments may be implemented by software, hardware, firmware, or any combination thereof. When the software is used to implement the embodiments, all or some of the embodiments may be implemented in a form of computer instructions. When the computer instructions are loaded and executed on the computer, all or some of the procedures or functions according to embodiments of the present invention are generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable or an optical fiber) or wireless (for example, infrared or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (Solid State Disk, SSD)), or the like.

Steps of the methods or algorithms described in embodiments of this application may be directly embedded into hardware, a software unit executed by a processor, or a combination thereof. The software unit may be stored in a RAM memory, a flash memory, a ROM memory, an EPROM memory, an EEPROM memory, a register, a hard disk, a removable magnetic disk, a CD-ROM, or a storage medium of any other form in the art. For example, the storage medium may be connected to a processor, so that the processor may read information from the storage medium and write information into the storage medium. Optionally, the storage medium may alternatively be integrated into the processor. The processor and the storage medium may be disposed in an ASIC, and the ASIC may be disposed in a terminal device. Optionally, the processor and the storage medium may alternatively be disposed in different components in the terminal device.

These computer instructions may alternatively be loaded to a computer or another programmable data processing device, so that a series of operations and steps are performed on the computer or the another programmable device, to generate computer-implemented processing. Therefore, the instructions executed on the computer or the another programmable device provide steps for implementing a specific function in one or more processes in the flowcharts and/or in one or more blocks in the block diagrams.

Although the present invention is described with reference to specific features and embodiments thereof, it is clear that various modifications and combinations may be made to the present invention without departing from the scope of the present invention. Correspondingly, the specification and the accompanying drawings are only example descriptions of the present invention defined by the appended claims, and are considered as any of or all modifications, variations, combinations, or equivalents that cover the scope of the present invention. It is clear that, a person skilled in the art can make various modifications and variations to the present invention without departing from the spirit and scope of the present invention. In this way, the present invention is intended to cover these modifications and variations of the present invention provided that these modifications and variations fall within the scope of the claims and their equivalent technologies.

**Claims**

1. A method for interpreting medical detection data, comprising:

   obtaining medical detection data to be interpreted, wherein the medical detection data to be interpreted comprises a detection image and indicator data;
   performing image recognition on the detection image, to obtain image recognition data; and interpreting, by using a rule library, the indicator data and the image recognition data, to obtain an interpretation result, wherein an interpretation rule in the rule library is obtained by mining and analyzing a plurality of pieces of interpreted data based on a data mining algorithm, and the rule library is dynamically updated with an interpretation process.

2. The method according to claim 1, wherein the method further comprises:

   mining and analyzing the plurality of pieces of interpreted data based on the data mining algorithm, to obtain the interpretation rule, wherein the interpreted data comprises indicator data, image recognition data, and an interpretation result obtained based on the indicator data and the image recognition data; and
   updating the rule library based on the interpretation rule.

3. The method according to claim 2, wherein the mining and analyzing the plurality of pieces of interpreted data based on the data mining algorithm, to obtain the interpretation rule comprises:

   classifying the plurality of pieces of interpreted data, to obtain interpreted data of a plurality of categories;
   selecting, from the interpreted data of the plurality of categories, interpreted data of a first category that meets a preset condition, wherein the preset condition is that a quantity of similar interpreted data in the interpreted data of the category is greater than a quantity threshold, interpretation results of any two pieces of similar interpreted data are the same, and indicator data of the two pieces of similar interpreted data falls within a same value range and/or image recognition data of the two pieces of similar interpreted data falls within a same value range; and
   obtaining the interpretation rule based on the similar interpreted data in the interpreted data of the first category.

4. The method according to any one of claims 1 to 3, wherein the detection image comprises a signal wave image, and the performing image recognition on the detection image, to obtain image recognition data comprises:
   performing image recognition on the signal wave image, to obtain at least one piece of attribute information of a signal wave waveform in the signal wave image.

5. The method according to any one of claims 1 to 4, wherein the interpreting, by using a rule library, the indicator data and the image recognition data, to obtain an interpretation result comprises:

   interpreting, by using the rule library, the image recognition data, to obtain at least two candidate interpretation results; and
   selecting the interpretation result from the at least two candidate interpretation results based on the indicator data.

6. The method according to claim 5, wherein there are at least two pieces of indicator data; and the selecting the interpretation result from the at least two candidate interpretation results based on the indicator data comprises:

   separately obtaining, based on value intervals of the at least two pieces of indicator data, candidate interpretation results corresponding to the corresponding value intervals, wherein the value intervals of the at least two pieces of indicator data are divided into a plurality of value intervals, and each value interval corresponds to at least one candidate interpretation result; and
   selecting, from all candidate interpretation results obtained based on the at least two pieces of indicator data, a candidate interpretation result that appears most frequently, to obtain the interpretation result.

7. The method according to claim 5, wherein the selecting the interpretation result from the at least two candidate interpretation results based on the indicator data comprises:

   obtaining target indicator data respectively corresponding to the at least two candidate interpretation results, wherein the target indicator data corresponding to any one candidate interpretation result is obtained based on indicator data in interpreted data corresponding to the candidate interpretation result;

separately determining similarities between the indicator data in the medical detection data and the target indicator data corresponding to the at least two candidate interpretation results; and

selecting a candidate interpretation result corresponding to a highest similarity, to obtain the interpretation result.

8. The method according to any one of claims 1 to 7, wherein before the interpreting, by using a rule library, the indicator data and the image recognition data, to obtain an interpretation result, the method further comprises:

determining, based on a quality control rule, that the indicator data and the image recognition data meet a preset quality control specification.

9. An apparatus for interpreting medical detection data, comprising:

a data obtaining module, configured to obtain medical detection data to be interpreted, wherein the medical detection data to be interpreted comprises a detection image and indicator data;

an image recognition module, configured to perform image recognition on the detection image, to obtain image recognition data; and

an interpretation module, configured to interpret, by using a rule library, the indicator data and the image recognition data, to obtain an interpretation result, wherein an interpretation rule in the rule library is obtained by mining and analyzing a plurality of pieces of interpreted data based on a data mining algorithm, and the rule library is dynamically updated with an interpretation process.

10. The apparatus according to claim 9, wherein the apparatus further comprises a data mining module, and the data mining module is configured to:

mine and analyze the plurality of pieces of interpreted data based on the data mining algorithm, to obtain the interpretation rule, wherein the interpreted data comprises indicator data, image recognition data, and an interpretation result obtained based on the indicator data and the image recognition data; and

update the rule library based on the interpretation rule.

11. The apparatus according to claim 10, wherein the data mining module is specifically configured to:

classify the plurality of pieces of interpreted data, to obtain interpreted data of a plurality of categories;

select, from the interpreted data of the plurality of categories, interpreted data of a first category that meets a preset condition, wherein the preset condition is that a quantity of similar interpreted data in the interpreted data of the category is greater than a quantity threshold, interpretation results of any two pieces of similar interpreted data are the same, and indicator data of the two pieces of similar interpreted data falls within a same value range and/or image recognition data of the two pieces of similar interpreted data falls within a same value range; and

obtain the interpretation rule based on the similar interpreted data in the interpreted data of the first category.

12. The apparatus according to any one of claims 9 to 11, wherein the detection image comprises a signal wave image, and the image recognition module is specifically configured to:

perform image recognition on the signal wave image, to obtain at least one piece of attribute information of a signal wave waveform in the signal wave image.

13. The apparatus according to any one of claims 9 to 12, wherein the interpretation module is specifically configured to:

interpret, by using the rule library, the image recognition data, to obtain at least two candidate interpretation results; and

select the interpretation result from the at least two candidate interpretation results based on the indicator data.

14. The apparatus according to claim 13, wherein there are at least two pieces of indicator data; and the interpretation module is specifically configured to:

separately obtain, based on value intervals of the at least two pieces of indicator data, candidate interpretation results corresponding to the corresponding value intervals, wherein the value intervals of the at least two pieces of indicator data are divided into a plurality of value intervals, and each value interval corresponds to at least one candidate interpretation result; and

select, from all candidate interpretation results obtained based on the at least two pieces of indicator data, a candidate interpretation result that appears most frequently, to obtain the interpretation result.

15. The apparatus according to claim 13, wherein the interpretation module is specifically configured to:

obtain target indicator data respectively corresponding to the at least two candidate interpretation results, wherein the target indicator data corresponding to any one candidate interpretation result is obtained based on indicator data in interpreted data corresponding to the candidate interpretation result;

separately determine similarities between the indicator data in the medical detection data and the target indicator data corresponding to the at least two candidate interpretation results; and select a candidate interpretation result corresponding to a highest similarity, to obtain the interpretation result.

16. The apparatus according to any one of claims 9 to 15, wherein before interpreting, by using the rule library, the indicator data and the image recognition data, to obtain the interpretation result, the interpretation module is further configured to:

determine, based on a quality control rule, that the indicator data and the image recognition data meet a preset quality control specification.

17. An electronic device, wherein the electronic device comprises one or more processors and one or more memories, the one or more memories store one or more computer instructions, and when the one or more computer instructions are executed by the one or more processors, the electronic device is enabled to perform the method according to any one of claims 1 to 8.

18. A computer-readable storage medium, wherein the computer-readable storage medium comprises computer instructions, and when the computer instructions are run on a computer, the computer is enabled to perform the method according to any one of claims 1 to 8.

19. A computer program product, wherein when a computer reads and executes the computer program product, the computer is enabled to perform the method according to any one of claims 1 to 8.

FIG. 1

FIG. 2

101

Data
obtaining
module

102

Image
recognition
module

103

Data
mining
module

301: Obtain a
plurality of pieces
of medical
detection data and
corresponding
interpretation
results

302: Output a
detection image
in the medical
detection data

303: Perform
image recognition,
to obtain image
recognition data

304: Output the
image
recognition data

305: Send a plurality of
pieces of interpreted data

306: Perform quality
control on the
plurality of pieces of
interpreted data

307: Mine and
analyze the data; and
update a rule library

FIG. 3

101                    102                    104

┌──────────┐    ┌──────────┐    ┌──────────────┐
│   Data   │    │  Image   │    │              │
│obtaining │    │recognition│    │Interpretation│
│  module  │    │  module  │    │   module     │
└──────────┘    └──────────┘    └──────────────┘

**401: Obtain medical detection data to be interpreted**

**402: Output a detection image in the medical detection data to be interpreted**

**403: Perform image recognition, to obtain image recognition data**

**404: Output the image recognition data**

**405: Output indicator data and the image recognition data**

**406: Perform quality control on the indicator data and the image recognition data**

**407: Interpret the indicator data and the image recognition data, to obtain an interpretation result**

FIG. 4

101

102

103

104

| Data obtaining module | Image recognition module | Data mining module | Interpretation module |

FIG. 5

Electronic device

602

601

Communication interface

Processor

603

Memory

FIG. 6

## INTERNATIONAL SEARCH REPORT

| International application No. |
|---|
| **PCT/CN2022/124928** |

**A. CLASSIFICATION OF SUBJECT MATTER**

G16H 50/20(2018.01)i

According to International Patent Classification (IPC) or to both national classification and IPC

**B. FIELDS SEARCHED**

Minimum documentation searched (classification system followed by classification symbols)

G16H

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 医疗, 检测, 数据, 判读, 图像, 指标, 识别, 规则, 挖掘, 分析, 更新, medical, detect, data, interpretation, image, quota, identify, rule, excavate, analyze, update

**C. DOCUMENTS CONSIDERED TO BE RELEVANT**

| Category* | Citation of document, with indication, where appropriate, of the relevant passages | Relevant to claim No. |
|---|---|---|
| Y | CN 111933279 A (JIANGSU RUIKANGCHENG MEDICAL TECHNOLOGY CO., LTD.) 13 November 2020 (2020-11-13)<br>    description, paragraph [0019] | 1-19 |
| Y | CN 113611403 A (ZHUHAI HAORUI TECHNOLOGY CO., LTD.) 05 November 2021 (2021-11-05)<br>    description, paragraph [0024] | 1-19 |
| A | CN 113297272 A (FUJIAN ZHONGRUI NETWORK CO., LTD.) 24 August 2021 (2021-08-24)<br>    entire document | 1-19 |
| A | CN 110781216 A (GUANGDONG UNIVERSITY OF TECHNOLOGY) 11 February 2020 (2020-02-11)<br>    entire document | 1-19 |

☐ Further documents are listed in the continuation of Box C.     ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **05 December 2022** | **15 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/124928**

| Patent document cited in search report | | | Publication date (day/month/year) | Patent family member(s) | Publication date (day/month/year) |
|---|---|---|---|---|---|
| CN | 111933279 | A | 13 November 2020 | None | |
| CN | 113611403 | A | 05 November 2021 | None | |
| CN | 113297272 | A | 24 August 2021 | None | |
| CN | 110781216 | A | 11 February 2020 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111540631